# EUROPEAN PATENT APPLICATION

(11) **EP 4 011 340 A1**
(43) Date of publication of application: **15.06.2022**
(21) Application number: 20867415.0
(22) Date of filing: 28.07.2020
(51) Int. Cl.: A61F 7/00

(54) **COLD AND HOT COMPRESS SYSTEM**

(30) Priority: 24.09.2019 CN 201910907746
(71) Applicant: Suzhou Microport Rehabtech (Group) Co., Ltd., Suzhou, Jiangsu 215000 (CN)
(72) Inventor: XIE, Fei, Suzhou, Jiangsu 215000 (CN); LUO, Yi, Suzhou, Jiangsu 215000 (CN); DI, Pei, Suzhou, Jiangsu 215000 (CN); YANG, Yiwei, Suzhou, Jiangsu 215000 (CN)
(74) Representative: Patentship Patentanwaltsgesellschaft mbH
(86) International application number: PCT/CN2020/105139
(87) International publication number: WO 2021/057240

(57) **Abstract**

A cold and/or hot compress system, comprising a water bag (101), an air bag (102), a water circulation assembly (2) in fluid communication with the water bag (101) and a pneumatic assembly (3) in fluid communication with the air bag (102). In the cold and/or hot compress system, the water circulation assembly (2) is independent from the pneumatic assembly (3). Therefore, during the delivery of a cooling or heating therapy, the independent pneumatic assembly (3) can be driven by an air pressure waveform defined by parameters pre-stored in a control panel (402) to cause the water bag (102) to apply cold/hot compress to a region of interest of a patient's body. Independent water and air circulation paths in the system avoid interference between water and air flows therein, allowing the system to applied more stable compress with minimized pressure errors. Moreover, a more constant water flow rate and a more uniform temperature distribution across the surface of the water bag are achievable.

## Description

### TECHNICAL FIELD

The present invention relates to the technical field of medical instruments and, more particularly, to a cold and/or hot compress system.

### BACKGROUND

Cold/hot compress is commonly applied to human and/or animal skin in daily life. Cold compress can locally cause contraction of blood vessels, slow blood flow, lower the permeability of capillaries, reduce exudation, slow down metabolism and effectively reduce the release of inflammatory mediators. Hot compress can promote blood circulation, enhance metabolism and expedite healing.

Cold compress, hot compress and deep vein thrombosis (DVT) compress are very common in clinical field. While the current practice in healthcare organizations remain basically the application of a heated or cooled towel to a wound, there have been introduced various cold/hot compress-related products into the marketplace, such as ice packs, ice compress devices and semiconductor refrigerators.

An ice pack can be chilled in a refrigerator or heated in a microwave oven and then applied to a wound. It is disadvantageous in being incapable of maintaining a constant desired cooling/heating temperature and having to be interchanged to maintain such a desired temperature.

An ice compress device simply transforms ice into cold water and pumps the water into a wrap bag for the purpose of circulating cooling. However, existing ice compress devices are associated with many disadvantages including 1) the need for replenishment of ice, 2) the mere capability of cooling, but the incapabilities of heating or alternate compress of cooling and heating, 3) the requirement of an external pneumatic assembly for transferring the cold water, which in turn imposes strict requirement on the air and water tightness of the used case and pipes, 4) only the adjustment of a cooling time being allowed and lack of other functions and 5) care that must be taken in the height where the device is disposed in order to avoid an insufficient water flow.

A semiconductor refrigerator works by heating or cooling water and causing it to flow through a semiconductor module using a pump and then introducing the hot or cold water into a wrap bag for the purpose of circulating cooling. Existing semiconductor refrigerators suffer from the following drawbacks: 1) the cooling temperature is uncontrollable, it is not allowed to choose between the cooling and heating modes, and the lowest achievable temperature depends on the ambient environment; 2) the cold or hot water is transferred pneumatically, creating fluctuations in the pressure used for cold compress and leading to an inconstant water flow rate; 3) it is not allowed to store and edit in advance a series of cold/hot compress therapy plans diversified in terms of treatment time, rest time, pulsating compression pressure and temperature in each cycle; 4) wrap bags have poor applicability, making the cold compress therapies only provide a limited range of body areas; 5) the devices for cooling or heating are not equipped with alarm functions compatible with the standard YY0709-2009 for alarm systems in medical electrical equipment and are thus not suitable for use in hospital wards that require long-term postoperative efficacy assessment involving cold compress without physician supervision; and 6) semiconductor cooling fans in the devices are noisy, disrupting patients' rest.

Therefore, it is necessary to have a cold and/or hot compress system with improved performance.

### SUMMARY OF THE INVENTION

It is an object of the present invention to overcome one or more of the above-described problems with the prior art by presenting a cold and/or hot compress system.

To achieve the above object, the present invention provides a cold and/or hot compress system comprising a water bag, an air bag, a water circulation assembly in fluid communication with the water bag and a pneumatic assembly in fluid communication with the air bag.

Preferably, the water circulation assembly comprises a water inlet pipe and a water outlet pipe and the pneumatic assembly comprises an air circulation pipe independent of the water inlet pipe and the water outlet pipe.

Preferably, the water circulation assembly comprises a water tank, a hydraulic pump and a water pathway.

The water bag is brought into communication with the water tank by the water outlet pipe, and the water tank, the hydraulic pump, the water pathway and the water bag are fluid communicatively connected sequentially in this order by the water inlet pipe. The water tank, the hydraulic pump, the water pathway, the water inlet pipe, the water bag, the water outlet pipe and the water tank are communicatively connected sequentially in this order, thus forming a hydraulic circuit.

Preferably, the water circulation assembly comprises a first semiconductor module and a second semiconductor module, which are disposed on opposing sides of the water pathway and connected to each other to form an electric current circuit so as to be able to exchange heat with the water pathway.

Preferably, the water circulation assembly comprises at least one heat sink in contact with the first semiconductor module and the second semiconductor module.

Preferably, a cooling fan is arranged on the side of the at least one heat sink away from the water pathway.

Preferably, the water circulation assembly comprises at least one vent window, and the cooling fan is fitted with the at least one heat sink on one side and with the at least one vent window on the other side.

Preferably, the pneumatic assembly comprises a pneumatic pump, a first solenoid valve and a second solenoid valve.

The air bag is connected to the pneumatic pump via the air circulation pipe, and the first solenoid valve is connected to the air bag via the air circulation pipe. The first solenoid valve is connected to the pneumatic pump and the second solenoid valve via the air circulation pipe.

Preferably, the pneumatic assembly comprises a mechanical relief valve connected to the first solenoid valve, the pneumatic pump and the second solenoid valve via the air circulation pipe.

Preferably, the cold and/or hot compress system further comprises a control device, wherein:
the control device comprises a display and interaction module and a control panel;
the display and interaction module is coupled to the control panel; and
the control panel is coupled to both the water circulation assembly and the pneumatic assembly and configured to communicate with the display and interaction module so as to enable the display and interaction module to provide an interface allowing interaction about multiple operating modes and parameters of the cold and/or hot compress system, the operating modes and parameters including one of run time of each cycle, rest time of each cycle, total run time, compression pressure parameters, choice of hot and cold compress modes and temperature settings, or a combination thereof.

Preferably, the cold and/or hot compress system further comprises at least one temperature sensor coupled to at least one of the water outlet pipe, the water inlet pipe, the first semiconductor module and the second semiconductor module.

Preferably, the water circulation assembly comprises a liquid level sensor connected to the water tank.

Preferably, the water circulation assembly comprises a first current loop detection sensor coupled to the hydraulic pump.

Preferably, the pneumatic assembly comprises an air pressure sensor connected to the first solenoid valve, the pneumatic pump, the second solenoid valve and the mechanical relief valve by the air circulation pipe.

Preferably, the pneumatic assembly comprises a second current loop detection sensor coupled to the pneumatic pump.

Preferably, the cold and/or hot compress system further comprises a wrap bag, which wraps both the water bag and the air bag that is disposed on one side of the water bag.

### BRIEF DESCRIPTION OF THE DRAWINGS

In the drawings, identical reference numbers identify similar elements or actions. The sizes and relative positions of the elements in the drawings are not necessarily drawn to scale. For example, the forms of the various elements and angles are not drawn to scale, and some of these elements are enlarged and located arbitrarily to improve the understanding of the drawing. In addition, the particular forms of the elements as drawn do not intend to convey any information concerning the real shape of the particular elements and only have been selected to facilitate its recognition in the drawings, wherein:
Fig. 1 depicts a schematic structural overview of a cold and/or hot compress system according to an embodiment of the present invention;
Fig. 2 depicts a schematic structural overview of semiconductor modules in the cold and/or hot compress system according to an embodiment of the present invention;
Fig. 3 is a schematic structural view of an independently-operating water circulation assembly according to an embodiment of the present invention; and
Fig. 4 is a schematic structural view of an independently-operating pneumatic assembly according to another embodiment of the present invention.

### DETAILED DESCRIPTION

Objects, features and advantages of the present invention will become more apparent upon reading the following more detailed description of the present invention in conjunction with the accompanying drawings. It should be understood that the specific embodiments disclosed herein are merely illustrative of, rather than limiting, the present invention.

As used herein, the terms "first", "second", "third" and the like are used to distinguish between similar elements and not necessarily for describing a specific sequential order.

A cold and/or hot compress system according to the claimed invention will be described in detail below with reference to the accompanying drawings.

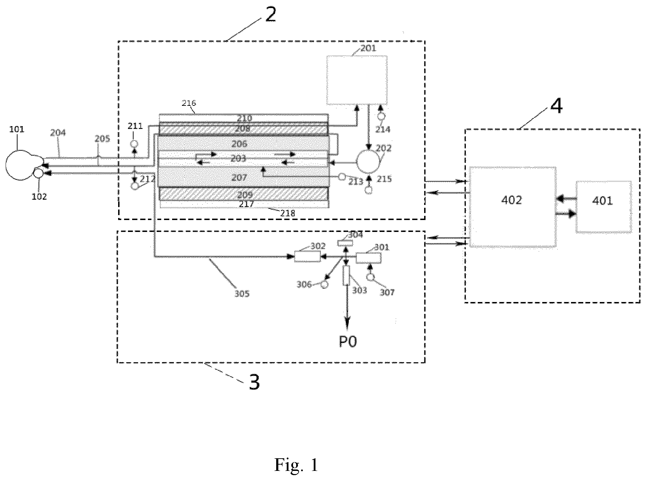

As shown in Fig. 1, the cold and/or hot compress system includes a water bag 101 for providing water to be circulated, an air bag 102 for providing air to be circulated, a water circulation assembly 2 in fluid communication with the water bag 101 and a pneumatic assembly 3 in fluid communication with the air bag 102.

The water circulation assembly 2 includes a water inlet pipe 205 and a water outlet pipe 204, and the pneumatic assembly 3 includes an air circulation pipe 305 independent from both the water inlet pipe 205 and the water outlet pipe 204.

The water circulation assembly 2 includes a water tank 201, a hydraulic pump 202 and a water pathway 203. The water outlet pipe 204 and the water inlet pipe 205 bring the water bag 101 into communication with the water tank 201. The water tank 201, the hydraulic pump 202, the water pathway 203 and the water bag 101 are fluid communicatively connected sequentially in this order by the water inlet pipe 205. In this way, the water tank 201, the hydraulic pump 202, the water pathway 203, the water inlet pipe 205, the water bag 101, the water outlet pipe 204 and water tank 201 are fluid communicatively connected sequentially in this order, thus forming a hydraulic circuit.

As would be appreciated by those skilled in the art, the water circulated in the water circulation assembly according to embodiments of the present invention refers to any liquid suitable for use in cold/hot compress, such as a mixture of water and other substance(s).

In some embodiments, the water circulation assembly 2 includes a first semiconductor module 206 and a second semiconductor module 207, which are disposed on opposing sides of the water pathway 203. Each of the first semiconductor module 206 and the second semiconductor module 207 is brought at one side into contact with the water pathway 203 so as to be able to exchange heat therebetween. In order to achieve increased heat exchange efficiency, the water pathway 203 may be U-shaped. Compared with conventional arrangements of semiconductor modules, arranging the first semiconductor module 206 and the second semiconductor module 207 on the opposing sides of the water pathway 203 overcomes the problem of excessive heat generated during cooling due to low heat dissipation efficiency of the system, which may lead to low cooling efficiency of the semiconductor modules and a too long time to be taken to achieve a cooled temperature of clinical significance and thus create inconvenience to both the patient and the physician. Arranging the first semiconductor module 206, the second semiconductor module 207 and the water pathway 203 in this way can result in improved cooling and heating efficiency, thus dispensing with the need for the use of a high power fan. Therefore, it can further solve the problems of excessive noise during operation, lack of operating convenience and the use of a noisy cooling fan for the semiconductor modules, which have been found to be associated with conventional hot and cold compress devices.

According to embodiments of the present invention, the cold and/or hot compress system operates by first adding pure water or another liquid with a high specific heat capacity to the water tank 201. As indicated by the arrows in Fig. 1, the liquid then flows from the water tank 201 through the hydraulic pump 202 into the water pathway 203. In this process, the first semiconductor module 206 and the second semiconductor module 207 arranged on the opposing sides of the water pathway 203 cool or heat the water to a preset temperature depending on the direction/amplitude of a current/voltage applied thereto. Finally, the heated or cooled water flows into the water bag 101 through the water inlet pipe 205 to heat or cool a region of interest of the patient's body. In this way, increased cooling or heating efficiency is achievable, dispensing with the need for the use of a high power fan in the system and thus reducing the system's overall noise generated during operation.

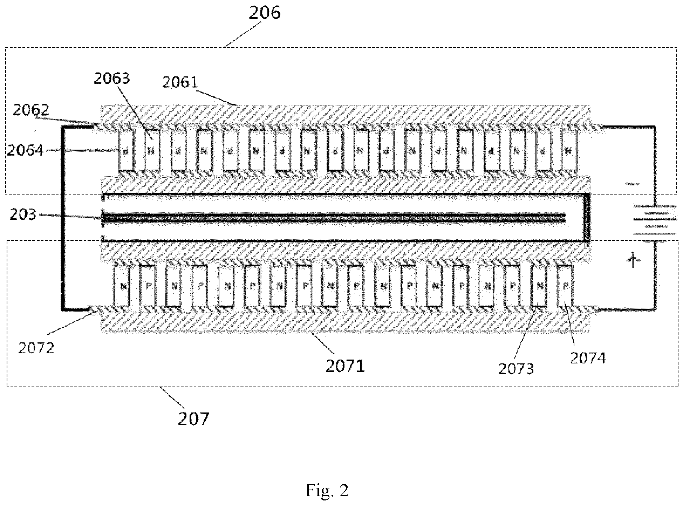

More specifically, the first semiconductor module 206 and the second semiconductor module 207 are structured as shown in Fig. 2. The first semiconductor module 206 includes a first insulating ceramic sheet 2061, a first copper plate 2062 and at least one pair of a first n-type semiconductor pellet 2063 and a first p-type semiconductor pellet 2064. The first insulating ceramic sheet 2061 is positioned on the side of the first semiconductor module 206 away from the water pathway 203. The first copper plate 2062 sequentially connects the alternately arranged first n-type 2063 and p-type 2064 semiconductor pellets in series. The first insulating ceramic sheet 2061 is connected to the first copper plate 2062. The second semiconductor module 207 includes a second insulating ceramic sheet 2071, a second copper plate 2072 and at least one pair of a second n-type semiconductor pellet 2073 and a second p-type semiconductor pellet 2074. The second insulating ceramic sheet 2071 is positioned on the side of the second semiconductor module 207 away from the water pathway 203. The second copper plate 2072 sequentially connects the alternately arranged second n-type 2073 and p-type 2074 semiconductor pellets in series. The second insulating ceramic sheet 2071 is connected to the second copper plate 2072. The first semiconductor module 206 and the second semiconductor module 207 are arranged in symmetry with respect to the water pathway 203. This can result in significantly improved cooling efficiency. Of course, in practice, the sizes of the first and second semiconductor modules may vary depending on an intended cooling effect, and they may be not necessarily arranged in symmetry.

According to an embodiment of the present invention, during operation of the semiconductor modules in the cold and/or hot compress system, as shown in Fig. 2, each pair of n-type and p-type semiconductor pellets is connected to form a couple, which, when energized by a DC current, will experience an energy transfer; when the current follows from the n-type semiconductor pellet to the p-type semiconductor pellet, the junction (on the outlet side of the water pathway 203) absorbs heat and becomes a cold side; and when the current flows from the p-type semiconductor pellet to the n-type semiconductor pellet, the junction releases heat and becomes a hot side. The amount of heat absorbed or released depends on the amplitude of the current and the number of pairs of n-type and p-type semiconductor pellets. A smaller temperature difference between the cold and hot sides will result in a better cooling effect.

In operation, as shown in Fig. 2, the first semiconductor module 206 and the second semiconductor module 207 are connected by wires to form an electric current circuit, and the liquid in the water pathway 203 can be heated or cooled through controlling the direction/amplitude of a current/voltage applied to the first semiconductor module 206 and the second semiconductor module 207. Arranging the first semiconductor module 206, the second semiconductor module 207 and the water pathway 203 in this way can result in improved cooling and heating efficiency, thus dispensing with the need for the use of a high power fan. Therefore, it can further solve the problems of excessive noise during operation, lack of operating convenience and the use of a noisy cooling fan for the semiconductor modules, which have been found to be associated with conventional hot and cold compress devices. The water pathway may be U-shaped. Alternatively, it may have a different shape such as linear or serpentine.

In some embodiments, the water circulation assembly 2 includes at least one heat sink in contact with the first semiconductor module 206 and/or the second semiconductor module 207. For example, in practice, as shown in Fig. 1, the water circulation assembly 2 may include a first heat sink 208 in contact with the first semiconductor module 206 and a second heat sink 209 in contact with the second semiconductor module 207. According to the present application, the semiconductor modules on the opposing sides can both dissipate heat, thus mitigating the problem of high operating noise associated with conventional semiconductor coolers/heaters and the problems of required replenishment of ice and incapability of continuous cooling for a long period of time arising from the use of conventional cold therapy devices. The number of the heat sink(s) may depend on the need of practical use.

In some embodiments, a cooling fan is arranged on the side of the at least one heat sink away from the water pathway 203. For example, in practical implementations, as shown in Fig. 1, a first cooling fan 210 may be arranged on the side of the first heat sink 208 away from the water pathway 203, and a second cooling fan 217 may be arranged on the side of the second heat sink 209 away from the water pathway 203. The first cooling fan 210 and the second cooling fan 217 may be implemented as low power fans, which can result in a reduced level of overall noise of the system during operation.

In some embodiments, the water circulation assembly 2 includes at least one vent window. In this case, the cooling fan is closely attached with the at least one heat sink on one side and is closely attached with the at least one vent window on the other side. For example, in practical implementations, as shown in Fig. 1, the water circulation assembly 2 may include a first vent window 216 and a second vent window 218. Additionally, the first cooling fan 210 may be closely attached with the first heat sink 208 on one side and closely attached with the first vent window 216 on the other side, and the second cooling fan 217 may be closely attached with the second heat sink 209 on one side and closely attached with the second vent window 218 on the other side. With the cooling fans closely attached with the respective heat sinks on one side and with the respective vent windows on the other side, flowing air can be introduced with an increased pressure to increase heat dissipation efficiency on the hot sides of the semiconductor modules. For example, suitable numbers of cooling fans and vent windows may be arranged at suitable distances apart, depending on the need of practical use.

In some embodiments, the pneumatic assembly 3 includes a pneumatic pump 301, a first solenoid valve 302 and a second solenoid valve 303. The air bag 102 is connected to the pneumatic pump 301 via the air circulation pipe 305. The first solenoid valve 302 is connected to the air bag 102 via the air circulation pipe 305. The first solenoid valve 302 is connected to the pneumatic pump 301 and the second solenoid valve 303 via the air circulation pipe 305.

In some embodiments, the pneumatic assembly 3 includes a mechanical relief valve 304, which is connected to the first solenoid valve 302, the pneumatic pump 301 and the second solenoid valve 303 via the air circulation pipe 305. The mechanical relief valve 304 is intended for protection in the event of an electrical control failure. It may be configured, when an air pressure in the air circulation pipe 305 exceeds a predetermined value, to be automatically opened to relieve the air pressure.

According to an embodiment, the cold and/or hot compress system further includes a control device 4, which includes a display and interaction module 401 and a control panel 402. The display and interaction module 401 is configured for display and interaction, and is able to edit and store various operating mode parameters of the cold and/or hot compress system. Specifically, the operating mode parameters may include, for example, one or combinations of "run time of each cycle", "rest time of each cycle", "total run time", "compression pressure parameters", "choice of cold compress, hot compress or cold/hot compress" and "temperature settings". The display and interaction module 401 may be coupled to the control panel 402, for example, by a wired or wireless connection, and configured for interaction and display of, for example, a pressure and temperature of the water, information about the current or voltage of the semiconductor modules, information about the pneumatic assembly, parameters for controlling the current/voltage of the semiconductor modules and/or air pressure parameters. The control panel 402 may be coupled to the water circulation assembly 2, for example, by a wired or wireless connection and may be configured to control the current/voltage of the semiconductor modules. The control panel 402 may be coupled to the pneumatic assembly 3, for example, by a wired or wireless connection and may be configured to set air pressure parameters for causing the pneumatic assembly 3 to provide a predefined air pressure waveform for driving the wrap bag to apply cold/hot compress to a region of interest of the patient's body.

In some embodiments, the water circulation assembly 2 includes a first temperature sensor 211 connected to the water outlet pipe 204. The first temperature sensor 211 is configured to monitor a temperature of cold/hot water flowing out of the water bag 101. The first temperature sensor 211 is further connected to the control panel 402.

In some embodiments, the water circulation assembly 2 includes a second temperature sensor 212 connected to the water inlet pipe 205. The second temperature sensor 212 is configured to monitor a temperature of cold/hot water flowing into the water bag 101. The second temperature sensor 212 is further connected to the control panel 402.

In some embodiments, the water circulation assembly 2 includes a third temperature sensor 213 connected to a contact interface between the first and second semiconductor modules 206, 207 and the water pathway 203. The third temperature sensor 213 is configured to monitor an actual heated or cooled temperature of the first semiconductor module 206 and the second semiconductor module 207. The third temperature sensor 213 is further connected to the control panel 402.

According an embodiment, the first temperature sensor 211, the second temperature sensor 212 and the third temperature sensor 213 in the cold and/or hot compress system are all connected to the control panel 402 so as to provide their sensed information to the control panel 402, which then processes the information and provides it to the display and interaction module 401. Based on the information displayed on the display and interaction module, a user may make an adjustment or take a control action. For example, the user may adjust a water temperature or the direction/amplitude of the current/voltage of the semiconductor modules.

In some embodiments, the water circulation assembly 2 includes a liquid level sensor 214 connected to the water tank 201. The liquid level sensor 214 is configured to monitor whether the liquid in the water tank 201 is in an amount within a desirable range. The liquid level sensor 214 is further connected to the control panel 402.

In some embodiments, the water circulation assembly 2 includes a first current loop detection sensor 215 coupled to the hydraulic pump 202. The first current loop detection sensor 215 is configured to monitor an operating condition of the hydraulic pump 202. The first current loop detection sensor 215 is further connected to the control panel 402.

In operation, based on temperatures of the water flowing into and out of the wrap bag that are monitored by the first and second temperature sensors 211, 212, the control panel 402 controls a temperature of the water circulating within the water bag 101 within a tolerance range of a preset temperature. The temperature monitored by the third temperature sensor 213 is displayed in real time as a reflection of an operating condition of the semiconductor modules. The control panel 402 determines, from data transmitted from the liquid level sensor 214, whether the monitored liquid level is appropriate, as well as whether it is necessary to raise an alarm. The first current loop detection sensor 215 is configured to monitor the operating condition of the hydraulic pump 202 and feed information thereabout to the control panel 402. If the control panel 402 finds that the hydraulic pump is not operating normally, it may cease the operation of the whole cold and/or hot compress system.

In some embodiments, the pneumatic assembly 3 includes an air pressure sensor 306, which is connected to the first solenoid valve 302, the pneumatic pump 301, the second solenoid valve 303 and the mechanical relief valve 304 by the air circulation pipe 305. The air pressure sensor 306 is further connected to the control panel 402.

In some embodiments, the pneumatic assembly 3 includes a second current loop detection sensor 307 coupled to the pneumatic pump 301. The second current loop detection sensor 307 is further coupled to the control panel 402. The second current loop detection sensor 307 is configured to monitor an operating condition of the pneumatic pump 301 and feed information thereabout to the control panel 402. If the control panel 402 finds that the pneumatic pump 301 is not operating normally, it may cease the operation of the whole compress system.

In operation, the air bag 102 may be connected to the pneumatic pump 301 via the air circulation pipe 305. The air circulation pipe 305 may be implemented as a hose. With the first solenoid valve 302 being opened and the second solenoid valve 303 being closed, the pneumatic pump 301 may fill an amount of air into the air bag 102 at a rate varying in real time to create an air pressure for compress, which varies in magnitude in a predefined triangular or trapezoidal waveform. As required by the waveform, both the first and second solenoid valves 302, 303 may be opened to relieve the pressure in the air bag 102 because the internal pressure in the air bag 102 is higher than the ambient atmospheric pressure. In order to enable pressure relief of the air bag 102 in predefined periods, a proper amount of air may be filled in the air bag 102 at the same time as pressure relief.

In some embodiments, the control panel 402 may be coupled to an external alarm and may cause the alarm to give an alarm when a received piece of sensor information indicates an abnormal condition. When this happens, the display and interaction module 401 coupled to the control panel 402 may display information about the fault. The control panel 402 may cause the external alarm coupled thereto to issue different alarms in response to faults of different classes. For one or more most severe fault classes, fault information may be displayed, and an audible indication may be provided at the same time in the form as specified in the standard YY0709-2009 for alarm systems in medical electrical equipment.

In an embodiment, the cold and/or hot compress system further includes a wrap bag, which wraps both the water bag 101 and the air bag 102 disposed external to the water bag 101. More specifically, the air bag 102 may be disposed farther away from the patient's body than the water bag 101, while the water bag 101 may be brought into contact with the patient's body. In this way, the air bag 102 can press the water bag 101 against the patient's body.

Alternatively, instead of providing the wrap bag, the water bag 101 may be attached to the air bag 102, for example, by binding (hook-and-loop fasteners).

In some embodiments, suitable non-limiting examples of the water outlet pipe 204 and the water inlet pipe 205 may include hoses that can insulate and preserve heat. In practical implementations, the water outlet pipe 204 and the water inlet pipe 205 may be both chosen as heat insulating hoses for cold/hot water in fluid communication with the water bag 101, the water tank 201, the hydraulic pump 202 and the water pathway 203.

In some embodiments, but without limitation, the air circulation pipe 305 may be implemented as a hose.

In some embodiments, the display and interaction module 401 includes a touch screen, which can be manipulated to set parameters of the system and perform other operations including causing the system to start or stop operation. In contrast to conventional semiconductor coolers/heaters that are inconvenient to operate and configure, the large-sized touch screen allows ease of manipulation by a user. The alarm scheme in compliance with the standard YY0709-2009 for alarm systems in medical electrical equipment allows long-term operation of the system for cooling, heating and compressing without the supervision of medical staff. During operation of the system in any of several operating modes including cooling, heating, cold compress, hot compress, alternate cooling and heating, and alternate cold and hot compress, the temperature sensors, the liquid level sensor, the current loop detection sensors, the air pressure sensor and the like can capture data indicating how well the operation is going on, and the control panel 402 can monitor and control properties of the components involved in the operation. In the event of any abnormal condition, the control panel 402 may provide an alarm compatible with the standard YY0709-2009 for alarm systems in medical electrical equipment, which calls for intervention of medical staff to prevent accidental damage to the patient's body or to the system. This solves the problem of long-term hot/cold compress without an alarm scheme compatible with the aforementioned standard, which may lead to safety issues.

In preferred embodiments, the water circulation assembly 2 and the pneumatic assembly 3 operate independently from each other.

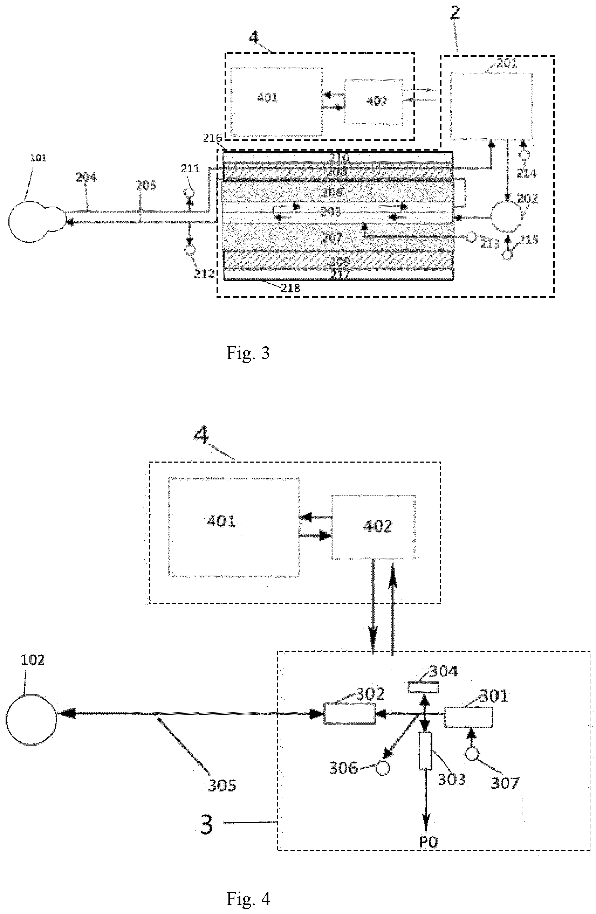

Fig. 3 is a structural schematic of the water circulation assembly 2 which operates independently according to an embodiment of the present invention. As shown, the water circulation assembly 2 includes the water bag 101, the water circulation assembly 2 and the control device 4, involved in water circulation.

The water circulation assembly 2 shown in Fig. 3 includes the water tank 201, the hydraulic pump 202, the water pathway 203, the water outlet pipe 204 and the water inlet pipe 205. The water tank 201, the hydraulic pump 202, the water pathway 203 and the water bag 101 are communicatively connected sequentially in this order by the water inlet pipe 205. The water bag 101 is brought into fluid communication with the water tank 201 by the water outlet pipe 204 and the water inlet pipe 205. The water tank 201, the hydraulic pump 202, the water pathway 203, the water inlet pipe 205, the water bag 101, the water outlet pipe 204 and the water tank 201 are communicatively connected sequentially in this order, thus forming a hydraulic circuit.

The water circulation assembly 2 shown in Fig. 3 further includes the first semiconductor module 206, the second semiconductor module 207, the first heat sink 208, the second heat sink 209, the cooling fan 210, the first vent window 216, the second cooling fan 217 and the second vent window 218. The first semiconductor module 206 and the second semiconductor module 207 are disposed on the opposing sides of the water pathway 203 in such a manner that they are brought into contact with the water pathway 203 on one side and respectively with the first heat sink 208 and the second heat sink 209 on the other side. The first cooling fan 210 is arranged on the side of the first heat sink 208 away from the water pathway 203, and the second cooling fan 217 is arranged on the side of the second heat sink 209 away from the water pathway 203. The first cooling fan 210 and the second cooling fan 217 are closely attached with the first heat sink 208 and the second heat sink 209, respectively, on one side, and are closely attached with the first vent window 216 and the second vent window 218, respectively, on the other side. With the cooling fans being closely attached with the respective heat sinks on one side and closely attached with the respective vent windows on the other side, flowing air can be introduced with an increased pressure to increase heat dissipation efficiency on the hot sides of the semiconductor modules.

The control device 4 shown in Fig. 3 includes the display and interaction module 401 and the control panel 402. The display and interaction module 401 is coupled to the control panel 402, for example, by a wired or wireless connection, and configured for interaction and display of, for example, a pressure and temperature of the water, information about the current and voltage of the semiconductor modules, and the like. The control panel 402 is coupled to the water circulation assembly 2 and configured to control the current or voltage of the semiconductor modules. The water circulation assembly 2 further includes the first temperature sensor 211, the second temperature sensor 212, the third temperature sensor 213, the liquid level sensor 214 and the first current loop detection sensor 215. The first temperature sensor 211 is coupled to the water outlet pipe 204 and configured to monitor a temperature of cold/hot water flowing out of the water bag 101. The second temperature sensor 212 is coupled to the water inlet pipe 205 and configured to monitor a temperature of cold/hot water flowing into the water bag 101. The third temperature sensor 213 is coupled to the contact interface between the first and second semiconductor modules 206, 207 and the water pathway 203, and configured to monitor an actual heated or cooled temperature of the first semiconductor module 206 and the second semiconductor module 207. The first temperature sensor 211, the second temperature sensor 212, the third temperature sensor 213, the liquid level sensor 214 and the first current loop detection sensor 215 are all coupled to the control panel 402 so as to communicate their sensed information to the control panel 402, which then processes the information and provides it to the display and interaction module 401. Based on the information displayed on the display and interaction module 401, a user may make an adjustment or take a control action. For example, he/she may adjust a water temperature or the direction/amplitude of the current/voltage of the semiconductor modules.

Fig. 4 is a structural schematic of the pneumatic assembly 3 which operates independently according to another embodiment of the present invention. As shown, the pneumatic assembly 3 includes the air bag 102, the pneumatic assembly 3 and the control device 4 that are involved in air circulation. The pneumatic assembly 3 includes the pneumatic pump 301, the first solenoid valve 302, the second solenoid valve 303 and the mechanical relief valve 304. The air bag 102 and the pneumatic pump 301 are interconnected by the air circulation pipe 305. The first solenoid valve 302 is connected to the air bag 102 via the air circulation pipe 305. The first solenoid valve 302 is connected to the pneumatic pump 301 and the second solenoid valve 303 by the air circulation pipe 305. The mechanical relief valve 304 is connected to the first solenoid valve 302, the pneumatic pump 301 and the second solenoid valve 303 by the air circulation pipe 305. The mechanical relief valve 304 is intended for protection in the event of an electrical control failure. It is configured, when an air pressure in the air circulation pipe 305 exceeds a predetermined value, to be automatically opened to relieve the air pressure.

The control device 4 shown in Fig. 4 includes the display and interaction module 401 and the control panel 402. The display and interaction module 401 is coupled to the control panel 402 and configured for interaction and display of information about the pneumatic assembly 3. The control panel 402 is coupled to the pneumatic assembly 3, and configured to set air pressure parameters for causing the pneumatic assembly 3 to provide a predefined air pressure waveform for driving the wrap bag to apply cold/hot compress to a region of interest of the patient's body.

The pneumatic assembly 3 shown in Fig. 4 includes the air pressure sensor 306 and the second current loop detection sensor 307. The air pressure sensor 306 is connected to the first solenoid valve 302, the pneumatic pump 301, the second solenoid valve 303 and the mechanical relief valve 304 by the air circulation pipe 305. The second current loop detection sensor 307 is coupled to the pneumatic pump 301. The air pressure sensor 306 and the second current loop detection sensor 307 are both coupled to the control panel 402. The second current loop detection sensor 307 is configured to monitor an operating condition of the pneumatic pump 301 and feed information thereabout to the control panel 402. If the control panel 402 finds that the pneumatic pump 301 is not operating normally, it may cease the operation of the whole compress system.

As shown in Fig. 4, the air bag 102 is connected to the pneumatic pump 301 by the air circulation pipe 305. The air circulation pipe 305 is implemented as a hose. With the first solenoid valve 302 being opened and the second solenoid valve 303 being closed, the pneumatic pump 301 may fill an amount of air into the air bag 102 at a rate varying in real time to create an air pressure for compress, which varies in magnitude in a predefined triangular or trapezoidal waveform. As required by the waveform, both the first and second solenoid valves 302, 303 may be opened to relieve the pressure in the air bag 102 because this internal pressure is higher than the ambient atmospheric pressure. In order to enable pressure relief of the air bag 102 in predefined periods, a proper amount of air may be filled in the air bag 102 at the same time as pressure relief.

In summary, the present invention provides a cold and/or hot compress system capable of providing more stable compress.

The cold and/or hot compress system includes a water bag 101, an air bag 102, a water circulation assembly 2 in fluid communication with the water bag 101 and a pneumatic assembly 3 in fluid communication with the air bag 102.

In some embodiments, the water circulation assembly 2 includes a water inlet pipe 205 and a water outlet pipe 204, and the pneumatic assembly 3 includes an air circulation pipe 305 that is independent from the water inlet pipe 205 and the water outlet pipe 204.

In some embodiments, the water circulation assembly 2 includes a water tank 201, a hydraulic pump 202 and a water pathway 203.

The water bag 101 is brought into communication with the water tank 201 by the water outlet pipe 204. The water tank 201, the hydraulic pump 202, the water pathway 203 and the water bag 101 are fluid communicatively connected sequentially in this order by the water inlet pipe 205. The water tank 201, the hydraulic pump 202, the water pathway 203, the water inlet pipe 205, the water bag 101, the water outlet pipe 204 and the water tank 201 are communicatively connected sequentially in this order, thus forming a hydraulic circuit.

In some embodiments, the water circulation assembly 2 includes a first semiconductor module 206 and a second semiconductor module 207, which are disposed on opposing sides of the water pathway 203.

In some embodiments, the water circulation assembly 2 includes at least one heat sink in contact with the first semiconductor module 206 and the second semiconductor module 207.

In some embodiments, a cooling fan is arranged on the side of the at least one heat sink away from the water pathway 203.

In some embodiments, the water circulation assembly 2 includes at least one vent window, and the cooling fan is closely attached with the at least one heat sink on one side and closely attached with the at least one vent window on the other side.

In some embodiments, the pneumatic assembly 3 includes a pneumatic pump 301, a first solenoid valve 302 and a second solenoid valve 303.

The air bag 102 is connected to the pneumatic pump 301 via the air circulation pipe 305, and the first solenoid valve 302 is connected to the air bag 102 via the air circulation pipe 305. The first solenoid valve 302 is connected to the pneumatic pump 301 and the second solenoid valve 303 via the air circulation pipe 305.

In some embodiments, the pneumatic assembly 3 includes a mechanical relief valve 304 connected to the first solenoid valve 302, the pneumatic pump 301 and the second solenoid valve 303 via the air circulation pipe 305.

In some embodiments, the cold and/or hot compress system further includes a control device 4.

The control device 4 includes a display and interaction module 401 and a control panel 402.

The display and interaction module 401 is coupled to the control panel 402.

The control panel 402 is coupled to both the water circulation assembly 2 and the pneumatic assembly 3.

In some embodiments, the cold and/or hot compress system further includes at least one temperature sensor coupled to the control panel 402. The at least one temperature sensor is further coupled to at least one of the water outlet pipe 204, the water inlet pipe 205, the first semiconductor module 206 and the second semiconductor module 207.

In some embodiments, the water circulation assembly 2 includes a liquid level sensor 214 connected to the water tank 201. The liquid level sensor 214 is further coupled to the control panel 402.

In some embodiments, the water circulation assembly 2 includes a first current loop detection sensor 215 coupled to the hydraulic pump 202. The first current loop detection sensor 215 is further coupled to the control panel 402.

In some embodiments, the pneumatic assembly 3 includes an air pressure sensor 306 connected to the first solenoid valve 302, the pneumatic pump 301, the second solenoid valve 303 and the mechanical relief valve 304 by the air circulation pipe 305. The air pressure sensor 306 is further coupled to the control panel 402.

In some embodiments, the pneumatic assembly 3 includes a second current loop detection sensor 307 coupled to the pneumatic pump 301. The second current loop detection sensor 307 is further coupled to the control panel 402.

In some embodiments, the cold and/or hot compress system further includes a wrap bag, which wraps both the water bag 101 and the air bag 102 that is disposed on one side of the water bag 101.

In some embodiments, but without limitation, the water outlet and inlet pipes are implemented as hoses that can insulate and preserve heat.

In some embodiments, but without limitation, the air circulation pipe is implemented as a hose.

The present invention provides a cold and/or hot compress system having higher performance than prior art ones and including a water bag, an air bag, a water circulation assembly in fluid communication with the water bag and a pneumatic assembly in fluid communication with the air bag. The present invention is advantageous over the prior art as follows:
1. The semiconductor modules in the water circulation assembly can both dissipate heat on the opposing sides, thus mitigating the problem of high operating noise associated with conventional semiconductor coolers/heaters and the problems of required replenishment of ice and incapability of continuous cooling for a long period of time arising from the use of conventional cold therapy devices.
2. The semiconductor modules arranged in symmetry with respect to the water pathway can heat or cool a liquid to a preset temperature, which then flows through the water circulation path into the water bag to ultimately heat or cool a region of interest of the patient's body. This results in greatly improved cooling or heating efficiency and overcomes the problem of excessive heat generated during cooling due to low heat dissipation efficiency of conventional semiconductor modules, which may lead to low cooling efficiency of the semiconductor modules and a too long time to be taken to achieve a cooled temperature of clinical significance and thus create inconvenience to both the patient and the physician.
3. The improved cooling or heating efficiency dispenses with the need for the use of a high power fan, resulting in reduced overall noise of the system during operation.
4. The use of the rate-adjustable pneumatic pump and the additional use of the solenoid valves overcome the problem of inconstant pressures or pressure waveforms during hot or cold compress arising from the use of conventional semiconductor coolers/heaters and allow the water bag to be better brought into contact with and fitted against the surface of a region of interest of the patient's body, resulting in a clinically improved cold/hot compress therapy effect.
5. Since the water circulation path is made independent from the air circulation path, interference between water and air flows therein is avoided, increasing constancy of the water flow rate, uniformity of the water bag's surface temperature and patient friendliness of the system.

According to embodiments of the present invention, the water circulation assembly and pneumatic assembly in the cold and/or hot compress system can operate independently of each other. Therefore, during the delivery of a cooling or heating therapy, the independent pneumatic assembly can be driven by an air pressure waveform defined by parameters pre-stored in the control panel to cause the water bag to apply cold or hot compress to an intended region of the patient's body. Since the water circulation path is made independent of the air circulation path, interference between water and air flows therein is avoided, allowing more stable compress to be applied by the system with minimized pressure errors. Moreover, constancy of the water flow rate, uniformity of the water bag's surface temperature and patient friendliness of the system can be increased. Further, in addition to the improved cooling or heating efficiency, the system of the present invention overall produces reduced noise during operation.

The various technical features of the foregoing embodiments may be combined in any way. Although not all such combinations have been described above for the sake of brevity, any of them is considered to fall within the scope of this specification as long as there is no contradiction between the technical features.

Presented above are merely several embodiments of the present application. Although these embodiments are described with some particularity and in some detail, it should not be construed that they limit the scope of the present application in any sense. Note that various variations and modifications can be made by those of ordinary skill in the art without departing from the concept of the present application. Accordingly, it is intended that all such variations and modifications are embraced within the scope of this application as defined in the appended claims.

## Claims

1. A cold and/or hot compress system, comprising a water bag (101), an air bag (102), a water circulation assembly (2) in fluid communication with the water bag (101) and a pneumatic assembly (3) in fluid communication with the air bag (102).

2. The cold and/or hot compress system according to claim 1, wherein the water circulation assembly (2) comprises a water inlet pipe (205) and a water outlet pipe (204), and wherein the pneumatic assembly (3) comprises an air circulation pipe (305) independent from the water inlet pipe (205) and from the water outlet pipe (204).

3. The cold and/or hot compress system according to claim 2, wherein the water circulation assembly (2) comprises a water tank (201), a hydraulic pump (202) and a water pathway (203),
wherein the water bag (101) is brought into communication with the water tank (201) by the water outlet pipe (204); the water tank (201), the hydraulic pump (202), the water pathway (203) and the water bag (101) is fluid communicatively connected sequentially by the water inlet pipe (205); the water tank (201), the hydraulic pump (202), the water pathway (203), the water inlet pipe (205), the water bag (101), the water outlet pipe (204) and the water tank (201) is communicatively connected sequentially to form a hydraulic circuit.

4. The cold and/or hot compress system according to claim 3, wherein the water circulation assembly (2) comprises a first semiconductor module (206) and a second semiconductor module (207), and wherein the first semiconductor module (206) and a second semiconductor module (207) are disposed on opposing sides of the water pathway (203) and connected to each other to form an electric current circuit so as to be configured to exchange heat with the water pathway.

5. The cold and/or hot compress system according to claim 4, wherein the water circulation assembly (2) comprises at least one heat sink in contact with the first semiconductor module (206) and the second semiconductor module (207).

6. The cold and/or hot compress system according to claim 5, wherein a cooling fan is arranged on the side of the at least one heat sink away from the water pathway (203).

7. The cold and/or hot compress system according to claim 6, wherein the water circulation assembly (2) comprises at least one vent window, and wherein the cooling fan is closely attached with the at least one heat sink on one side and closely attached with the at least one vent window on the other side.

8. The cold and/or hot compress system according to claim 2, wherein the pneumatic assembly (3) comprises a pneumatic pump (301), a first solenoid valve (302) and a second solenoid valve (303),
wherein the air bag (102) is connected to the pneumatic pump (301) via the air circulation pipe (305); the first solenoid valve (302) is connected to the air bag (102) via the air circulation pipe (305); the first solenoid valve (302) is connected to the pneumatic pump (301) and the second solenoid valve (303) via the air circulation pipe (305).

9. The cold and/or hot compress system according to claim 8, wherein the pneumatic assembly (3) comprises a mechanical relief valve (304), and wherein the mechanical relief valve (304) is connected to the first solenoid valve (302), the pneumatic pump (301) and the second solenoid valve (303) via the air circulation pipe (305).

10. The cold and/or hot compress system according to any one of claims 1 to 9, further comprising a control device (4),
the control device (4) comprising a display and interaction module (401) and a control panel (402);
the display and interaction module (401) coupled to the control panel (402),
wherein the control panel (402) is coupled to both the water circulation assembly (2) and the pneumatic assembly (3) and configured to communicate with the display and interaction module so as to enable the display and interaction module to provide an interface allowing interaction about multiple operating modes and parameters of the cold and hot compress system; the operating modes and parameters include one or combinations of: run time of each cycle, rest time of each cycle, total run time, compression pressure parameters, choice of hot and cold compress modes and temperature settings.

11. The cold and/or hot compress system according to any one of claims 4 to 7, further comprising at least one temperature sensor connected to at least one of the water outlet pipe (204), the water inlet pipe (205), the first semiconductor module (206) and the second semiconductor module (207).

12. The cold and/or hot compress system according to any one of claims 3 to 7, wherein the water circulation assembly (2) comprises a liquid level sensor (214) connected to the water tank (201).

13. The cold and/or hot compress system according to any one of claims 3 to 7, wherein the water circulation assembly (2) comprises a first current loop detection sensor (215) connected to the hydraulic pump (202).

14. The cold and/or hot compress system according to any one of claims 8 to 9, wherein the pneumatic assembly (3) comprises an air pressure sensor (306), and wherein the air pressure sensor (306) is connected to the first solenoid valve (302), the pneumatic pump (301), the second solenoid valve (303) and the mechanical relief valve (304) by the air circulation pipe (305).

15. The cold and/or hot compress system according to any one of claims 8 to 9, wherein the pneumatic assembly (3) comprises a second current loop detection sensor (307) connected to the pneumatic pump (301).

16. The cold and/or hot compress system according to any one of claims 1 to 9, further comprising a wrap bag, and wherein the wrap bag wraps both the water bag (101) and the air bag (102) that is disposed on one side of the water bag (101).
